(19) Europäisches Patentamt
European Patent Office
Office européen des brevets

(11) Veröffentlichungsnummer : **0 059 688**
**B1**

(12) # EUROPÄISCHE PATENTSCHRIFT

(45) Veröffentlichungstag der Patentschrift : **15.05.85**

(51) Int. Cl.⁴ : **C 07 D237/04**, A 61 K 31/535

(21) Anmeldenummer : **82810090.9**

(22) Anmeldetag : **26.02.82**

(54) **Pyridazinone, Verfahren zu ihrer Herstellung, pharmazeutische Präparate enthaltend diese Verbindungen und deren Verwendung.**

(30) Priorität : **04.03.81 CH 1443/81**

(43) Veröffentlichungstag der Anmeldung : **08.09.82 Patentblatt 82/36**

(45) Bekanntmachung des Hinweises auf die Patenterteilung : **15.05.85 Patentblatt 85/20**

(84) Benannte Vertragsstaaten : **AT BE CH DE FR IT LI LU NL SE**

(56) Entgegenhaltungen :
**DE-A- 2 207 517**
**DE-A- 2 854 191**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Göschke, Richard, Dr.**
**Felixhägilstrasse 21**
**CH-4103 Bottmingen (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 059 688 B1

Jouve, 18, rue St-Denis, 75001 Paris, France

**Beschreibung**

Die vorliegende Erfindung betrifft neue Pyridazinone, insbesondere 4,5-Dihydro-3(2H)-pyridazinoe, deren Herstellung, sowie pharmazeutische Präparate, die diese neuen Verbindungen enthalten, und auch ihre Verwendung.

In der DE-A-2 207 517 werden unter anderem im Phenylrest in p-Stellung durch eine Heterocyclus substituierte 6-Phenyl-4,5-dihydro-3(2H)-pyridazinone, welche eine anti-hypertensive Wirkung aufweisen und in der DE-A-2 854 191 werden in p-Stellung durch eine im Alkylrest polyhalogenierte Acylaminogruppe substituierte 6-Phenyl-4,5-dihydro-3(2H)-pyridazinone beschrieben, welche sich durch eine thrombocytenaggregationshemmende und blutdrucksenkende Eigenschaft auszeichnen.

Ueberraschenderweise wurde nun gefunden, dass die neuen Verbindungen der allgemeinen Formel I

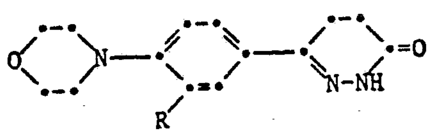

(I)

worin R ein Halogenatom, die Amino-, Acetylamino-, Methyl-, Cyano-, Methoxy oder die Trifluormethylgruppe bedeutet, und ihre tautomeren Formen eine ausgeprägte antithrombotische Wirkung aufweisen.

Halogenatome sind in erster Linie Chlor- oder Bromatome, können aber auch Fluor- oder Jodatome sein.

Die Verbindungen der Formel I weisen wertvolle pharmakologische Eigenschaften auf. So besitzen sie beispielsweise ausgeprägte antithrombotische Wirksamkeit. Diese kann beispielsweise am Meerschweinchen anhand der Hemmung der Thrombocytopenie nach Induktion durch ADP (Thromboembolism, Edited by J. R. Mitchell & J. G. Doment, Academic Press, (1977), p. 36) im Dosisbereich von etwa 30 bis 300 mg/kg p. o., anhand der Forssman-Reaktion (Thrombosis, Haemostasis 42, 100 (1979)) im Dosisbereich von etwa 100 bis 300 mg/kg p. o., und anhand der Hemmung der an einem Baumwollfaden sich bildenden Thrombose in einem extracorporaelmen shunt and der Ratte (Methode analog Brit. J. Pharmacol., *73*, 219 P (1981) im Dosisbereich von etwa 5 bis 50 mg/kg. p. o., sowie anhand der Hemmung der ex vivo durch Collagen oder Arachindonsäure induzierten Plättchenaggregation nach vorausgegangener peroralen Applikation des Wirkstoffes in Dosen von 50 bis 300 mg/kg nachgewiesen werden. Die Verbindungen der allgemeinen Formel I sind dementsprechend vorzüglich geeignet zur Behandlung thrombotischer Erkrankungen und können als Wirkstoffe in antithrombotischen Arzneimitteln verwendet werden.

Von besonderem Interesse in dieser Erfindung sind Verbindungen, in denen R die Bedeutung eines Chlor- oder Bromatomes haben. Besonders zu nennen ist das 6-(4-Morpholino-3-chlorphenyl)-4,5-dihydro-3(2H)-pyridazinon. Des weiteren von besonderem Interesse sind Verbindungen der Formel I, in denen R eine Amino-, Acetylamino- oder Cyanogruppe bedeutet. Des weiteren sind noch besonders zu nennen das 6-(3-Amino-4-morpholino-phenyl)-4,5-dihydro-3(2H)-pyridazinon, das 6-(3-Acetamino-4-morpholino-phenyl)-4,5-dihydro-3(2H)-pyridazinon und das 6-(3-Cyan-4-morpholino-phenyl)-4,5-dihydro-3(2H)-pyridazinon.

Die neuen Pyridazinone der Formel I werden nach an sich bekannten Methoden hergestellt.

So kann man die neuen Verbindungen der Formel I erhalten, indem man eine Ketocarbonsäure der Formel II

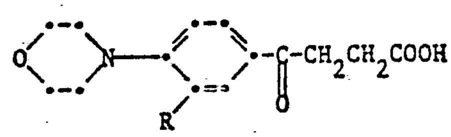

(II)

oder ein reaktionsfähiges Derivat einer solchen Ketocarbonsäure mit Hydrazin umsetzt. Vorzugsweise verwendet man das in Hydratform vorliegende Hydrazinhydrat, welches im Ueberschuss verwendet auch gleichzeitig als Lösungsmittel dienen kann. Zweckmässiger ist es jedoch, ein zusätzliches Lösungsmittel zuzusetzen. Als inerte Lösungsmittel eignen sich vorzugsweise Alkohole wie z. B. Methanol, Aethanol, Isopropanol, n-Butanol, Isoamylalkohol, Glykole und deren Aether wie Aethylenglykol, Diäthylenglykol, Aethylenglykolmonomethyl- oder -monoäthyläther (Methylglykol oder Aethylglykol), ferner Aether, insbesondere wasserlösliche Aether wie Tetrahydrofuran, Dioxan oder Aethylenglykoldimethyläther (Diglyme) ; ferner Wasser, sowie Gemische dieser Lösungsmittel untereinander, insbesondere Gemische mit Wasser, z. B. wässriges Aethanol. Die Reaktionstemperaturen liegen zweckmässig zwischen etwa 20 und etwa 200 °C, vorzugsweise zwischen 60 und 80 °C.

Als reaktionsfähige Derivate der Ketocarbonsäure der Formel II eignen sich beispielsweise die Ester, insbesondere Niederalkylester, wie z. B. Methyl- oder auch Aethylester. Ferner können noch die Säureamide und Säurehalogenide der Säuren der Formel II, insbesondere die Säurechloride oder Säurebromide verwendet werden. Weitere geeignete reaktionsfähige Derivate der Carbonsäure der

formel II können während der Reaktion in situ gebildet werden. Dazu gehören beispielsweise die Hydrazone der Formel $R_1—C(=N—NH_2)—CH_2—CH_2—COOH$, die Hydrazide der Formel $R_1—CO—CH_2—CH_2—CO—NH—NH_2$ und die Hydrazone der Hydrazide der Formel

$$R_1—C(=N—NH_2)—CH_2—CH_2—CO—NH—NH_2$$

worin $R_1$ die Bedeutung des Restes

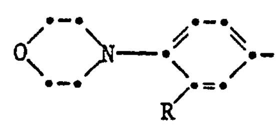

hat.

Die *in situ* gebildeten Ausgangsstoffe werden aus den Ketocarbonsäuren der Formel II hergestellt und werden aus dem Reaktionsgemisch nicht isoliert, sondern werden weiter zu den Verbindungen der Formel I umgesetzt.

Die Verbindungen der Formel I können auch erhalten werden, indem man Verbindungen der Formel III

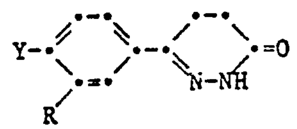

(III)

worin R die unter der Formel I angegebene Bedeutung hat, und Y eine zusammen mit Wasserstoff abspaltbare Gruppe Bedeutet, mit Morpholin umsetzt. Das verwendete Morpholin wird vorteilhafterweise als freie Base im Ueberschuss eingesetzt, kann jedoch auch in Form eines Säureadditionssalzes, beispielsweise als Hydrohalogenid, wie z. B. Hydrochlorid, verwendet werden.

Bei Verwendung eines nur geringen Ueberschusses des Morpholins als freie Base oder bei Verwendung des Morpholins als Säureadditionssalz ist es zweckmässig, eine stöchiometrisch äquivalente Menge z. B. eines tertiären Alkylamins, wie Triäthylamin oder N-Aethyldiisopropylamin, zusätzlich hinzuzusetzen.

Die beschriebene Umsetzung von Verbindungen der Formel III mit Morpholin erfolgt gegebenenfalls in Gegenwart eines Lösungsmittels, vorzugsweise eines aprotischen Lösungsmittels. Beispiele für bevorzugt verwendbare Lösungsmittel sind Aether, wie z. B. Diäthyläther und Tetrahydrofuran, insbesondere aliphatische Ketone und Ester, wie z. B. Aceton, Methyläthylketon und Aethylacetat, aromatische Kohlenwasserstoffe, wie z. B. Benzol, Toluol oder Xylol, sowie Acetonitril. Besonders bevorzugt kann die Umsetzung in Diäthyläther oder Acetonitril ausgeführt werden.

Die Umsetzungen können bei einer Temperatur zwischen 0-150 °C, vorzugsweise jedoch zwischen Raumtemperatur und Rückflusstemperatur des Reaktionsgemisches ausgeführt werden.

Eine zusammen mit Wasserstoff abspaltbare Gruppe Y ist z. B. in erster Linie eine freie oder vorzugsweise verätherte Mercaptogruppe, ferner eine gegebenenfalls reaktionsfähige, funktionell abgewandelte Hydroxygruppe oder die Nitroaminogruppe. Eine verätherte Mercaptogruppe ist in erster Linie eine durch einen gegebenenfalls substituierten Kohlenwasserstoffrest, insbesondere aliphatischen Charakters, verätherte Mercaptogruppe. Sie stellt in erster Linie Niederalkylthio, z. B. Methylthio, Aethylthio oder Butylthio oder Phenylniederalkylthio, z. B. Phenylthio oder Benzylthio dar. Eine gegebenenfalls reaktionsfähige, funktionell abgewandelte Hydroxygruppe ist eine freie und u. a. eine entsprechende veresterte Hydroxygruppe. Eine solche ist u. a. Halogen, z. B. Chlor oder Brom, oder Niederalkylsulfonyloxy, z. B. Methansulfonyloxy.

Vorzugsweise bedeutet Y als eine zusammen mit Wasserstoff abspaltbare Gruppe ein Halogenatom, wie z. B. Chlor oder Brom.

Verbindungen der allgemeinen Formel I können auch hergestellt werden, indem man eine Verbindung der Formel IV

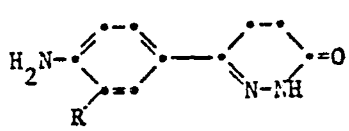

(IV)

mit einem Diäthylätherderivat der Formel V

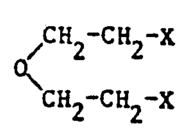

(V)

worin X eine zusammen mit Wasserstoff abspaltbare Gruppe bedeutet, umsetzt.

Die Umsetzung einer Verbindung der Formel IV mit einer Verbindung der Formel V erfolgt vorzugsweise in Gegenwart einer organischen Base wie z. B. eines tertiären Alkylamins, wie Triäthylamin oder N-Aethyldiisopropylamin.

Die beschriebene Umsetzung von Verbindungen der Formel IV mit einer Verbindung der Formel V erfolgt gegebenenfalls in Gegenwart eines Lösungsmittels, vorzugsweise eines polaren Lösungsmittels, wie z. B. Dimethylformamid.

Die Umsetzungen können bei einer Temperatur zwischen 0-200 °C, vorzugsweise jedoch zwischen Raumtemperatur und Rückflusstemperatur des Reaktionsgemisches ausgeführt werden.

Eine zusammen mit Wasserstoff abspaltbare Gruppe X ist bereits weiter oben unter Formel III für das Symbol Y definiert worden.

Vorzugsweise bedeutet X als eine zusammen mit Wasserstoff abspaltbare Gruppe ein Halogenatom, wie z. B. Chlor oder Brom.

Nach einem weiteren Verfahren können Verbindungen der Formel I, worin R ein Halogenatom oder die Cyanogruppe bedeutet, hergestellt werden, indem man eine Verbindung der Formel VI

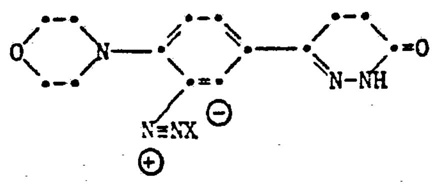

(VI)

worin $X^{\ominus}$ ein Anion einer Mineralsäure bedeutet, beispielsweise in Gegenwart von Kupfer oder eines Kupfer-I-salzes, beispielsweise eines Halogenids oder Cyanids erhitzt. Ein Anion einer Mineralsäure ist beispielsweise das Anion einer Halogenwasserstoffsäure. Im Falle der Einführung von R = Fluor ist $X^{\ominus}$ ein Fluorid- oder Tetrafluorboratanion. Die Erhitzung einer Verbindung der Formel VI erfolgt im Falle, wenn X ein Fluorid- oder Tetrafluorboratanion ist, in Flussäure oder in Tetrafluorborsäure. Bei der Einführung einer Cyanogruppe setzt man beispielsweise das Diazoniumsalz der Formel VI mit Kupfer-I-cyanid, das in Kaliumcyanid komplex gelöst ist, um. Beispielsweise setzt man ein Diazoniumsalz der Formel VI mit einem Gemenge aus Kaliumcyanid und Kupfer-I-sulfates um. Die Freisetzung des Diazoniumsalzes erfolgt thermisch bei Temperaturen zwischen 30-150 °C, vorzugsweise zwischen 30-40° bei Vorliegen einez Diazoniumfluorids und zwischen 100-150 °C bei Vorliegen eines Diazoniumtetrafluorborates.

Die Diazotierung aromatischer Amine erfolgt beispielsweise mit einem Alkalimetall-, wie z. B. Natriumnitrit, vorzugsweise mit Hilfe von trockenem Natriumnitrit. Die Diazotierung wird beispielsweise bei einer Temperatur zwischen (−) 10 bis (+) 10 °C, vorzugsweise bei einer Temperatur zwischen 0-5 °C ausgeführt. Durch Umsetzung mit einer Mineralsäure erhält man Verbindungen der Formel VI, in der X ein Anion einer Mineralsäure bedeutet.

Nach einem weiteren Verfahren können Verbindungen der Formel I, worin R ein Halogenatom bedeutet, hergestellt werden, indem man Verbindungen der Formel VII

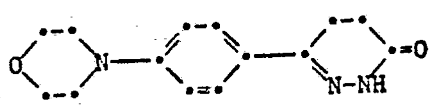

(VII)

halogeniert.

Die Halogenierung kann einerseits unter Verwendung von Halogen, vorzugsweise in Gegenwart einer Lewissäure, z. B. eines Eisen-III-, Aluminium-, Antimon-III- oder Zinn-IV-halogenids ausgeführt werden. Die Halogenierung kann andererseits auch mit Hilfe eines Halogenüberträgers, wie z. B. in Gegenwart eines Schwermetalls, beispielsweise Eisen oder unter Verwendung eines Halogenierungsmittels, wie z. B. Chlorwasserstoff in Gegenwart eines Oxydationsmittels, wie z. B. Wasserstoffperoxyd, oder eines Alkalimetall-, z. B. Natriumchlorats, eines Nitrosylhalogenids, z. B. Nitrosylchlorids oder -bromids oder eines N-Halogen-imids, z. B. Bromsuccinimids oder -phthalimid, ausgeführt werden.

Die direkte Einführung eines Jodatoms erfolgt bei Verwendung von Jodwasserstoff in Gegenwart eines Oxydationsmittels, beispielsweise in Gegenwart von Salpetersäure oder Quecksilberoxyd.

Die erwähnten Halogenierungen werden je nach Bedeutung des Halogenatoms bei Temperaturen zwischen − 10 °C und Rückflusstemperatur des Reaktionsgemisches, vorzugsweise bei Temperaturen zwischen − 5° und 30 °C ausgeführt.

Verbindungen der Formel I, in der R eine freie Aminogruppe bedeutet, können auch erhalten werden, indem man eine Verbindung der Formel VIII

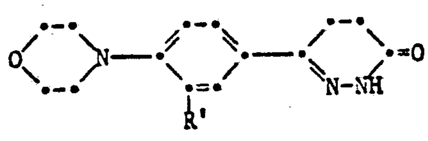

(VIII)

in der R' durch eine Schutzgruppe geschützte Aminogruppe bedeutet, solvolysiert.

Eine den Aminorest schützende Gruppe bzw. eine Aminoschutzgruppe ist in erster Linie eine Acylgruppe, wie Acyl einer aliphatischen, aromatischen oder araliphatischen Carbonsäure, insbesondere Niederalkanoyl, z. B. Acetyl oder Propionyl, oder Aroyl, z. B. Benzoyl, oder Acyl der Ameisensäure oder eines Kohlensäurehalbderivates, z. B. -esters,wie Formyl, Niederalkoxycarbonyl, z. B. Aethoxycarbonyl oder tert.-Butyloxycarbonyl, oder Arylniederalkoxycarbonyl, z. B. Benzyloxycarbonyl.

Die Abspaltung eines als Aminoschutzgruppe verwendeten Acylrestes kann in an sich bekannter Weise, z. B. durch Solvolyse, in erster Linie mittels Alkoholyse, ferner mittels Hydrolyse erfolgen. Die alkoholytische Abspaltung eines Acylrestes kann z. B. in Gegenwart eines stark basischen Mittels, bei erhöhter Temperatur, z. B. bei etwa 50 °C bis etwa 120 °C, erfolgen. Dabei verwendet man insbesondere ein Niederalkanol, z. B. n-Butanol oder Aethanol, und als starke Base ein Alkalimetall-, z. B. Natrium- oder Kalium-niederalkanolat, z. B. -n-butylat oder -äthylat, oder ein Alkalimetall-hydroxid, z. B. Natrium- oder Kaliumhydroxid.

Aminoschutzgruppen, beispielsweise Niederalkoxycarbonylgruppen, wie tert.-Butyloxycarbonyl, lassen sich besonders schonend acidolytisch, z. B. durch Behandeln mit Trifluoressigsäure, abspalten. Eine weitere, besonders schonend abspaltbare Aminoschutzgruppe ist eine Aethoxycarbonylgruppe, die in β-Stellung eine mit drei Kohlenwasserstoffresten substituierte Silylgruppe, wie Triphenylsilyl-, Dimethyl-butyl-silyl oder vor allem Trimethylsilylgruppe, trägt. Eine solche β-(Trimethylsilyl)-äthoxycarbonylgruppe bildet mit der zu schützenden Aminogruppe eine entsprechende β-Trimethylsilyläthoxycarbonylaminogruppe, welche sich unter milden Bedingungen durch Einwirkung von Fluoridionen abspalten lässt. Als Fluoridionen-abgebende Reagentien kommen beispielsweise Fluoride quaternärer organischer Basen, wie Tetraäthylammoniumfluorid in Frage.

Es ist dabei zu beachten, dass als Aminoschutzgruppe nur solche in Frage kommen, die selektiv unter Erhaltung der gewünschten Struktur der Verbindungen der allgemeinen Formel I abspaltbar sind.

Eine Verbindung der Formel I, in der R eine Aminogruppe bedeutet, kann man auch erhalten, indem man in einer Verbindung der Formel IX

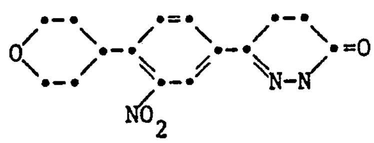

(IX)

die Nitrogruppe zur Aminogruppe selektiv reduziert. Reduktionen werden vorzugsweise katalytisch, beispielsweise in Gegenwart eines Edelmetallkatalysators, wie z. B. Platin oder auch Palladium auf Kohle mittels Wasserstoff ausgeführt. Reduktionen können jedoch auch nach dem weiter oben beschriebenen methoden, beispielsweise mit Zinn und Salzsäure oder auch Zinn(II)-chlorid erfolgen, wobei jedoch die katalytische oben erwähnte Methode bevorzugt wird.

In erhaltenen Verbindungen der Formel I kann man im Rahmen der Definition der Endprodukte Substituenten einführen, abwandeln oder abspalten.

Beispielsweise lassen sich erhaltene Verbindungen der Formel I, in der R eine freie Aminogruppe bedeutet, mit Hilfe eines Acylierungsmittels, beispielsweise eines Acetylhalogenids oder auch Essigsäureanhydrid in Verbindungen der Formel I unwandeln, in der R die Bedeutung einer Acetylaminogruppe hat. Die Acylierung wird vorzugsweise in Gegenwart einer organischen Base wie zum Beispiel in Gegenwart von Pyridin oder auch eines tertiären Alkylamins, wie Triäthylamin oder N-Aethyldiisopropylamin ausgeführt.

Die beschriebenen Verfahren können in gewohnter Weise bei Raumtemperatur, unter Kühlen oder Erwärmen, bei Normaldruck oder erhöhtem Druck, und, wenn notwendig, in Gegenwart oder Abwesenheit eines Verdünnungsmittels, Katalysators oder Kondensationsmittels ausgeführt werden. Wenn notwendig, können die Umsetzungen auch in der Atmosphäre eines inerten Gases, wie z. B. Stickstoff, erfolgen.

Die Ausgangsstoffe sind bekannt, oder falls sie neu sind, lassen sich nach an sich bekannten Methoden herstellen. Wo es sich als zweckdienlich erweist, sind die verwendeten Ausgangsprodukte im Anschluss an das beschriebene Verfahren bereits beschrieben worden.

Ketocarbonsäuren der Formel II können nach an sich bekannten Methoden hergestellt werden, beispielsweise für Verbindungen der Formel II, in denen R ein Halogenatom oder auch eine Cyanogruppe bedeutet, durch Halogenierung oder Einführung einer Cyanogruppe in einer Verbindung der Formel X

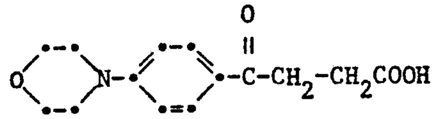

(X)

nach den oben beschriebenen Methoden, die in analoger Weise ausgeführt werden.

Verbindungen der allgemeinen Formel III sind bekannt oder können nach den von J. D. Albright et al,

J. Het. Chem. *15*, 881 (1978) beschriebenen Methode aus der entsprechenden Ketocarbonsäure durch Umsetzung mit Hydrazin erhalten werden. In analoger Weise können auch die Ausgangsverbindungen der allgemeinen Formel IV aus den entsprechenden Aminoketocarbonsäuren erhalten werden. Auch die Ausgangsverbindungen der allgemeinen Formel V sind bekannt und können beispielsweise aus den entsprechenden Dihydroxydiäthyläthern durch Veresterung mit einer Säure, beispielsweise Halogenwasserstoffsäure erhalten werden. Diäthylätherderivate der Formel V lassen sich auch aus entsprechend substituierten Alkoholen der Formel $OH—CH_2—CH_2—X$ durch Verätherung erhalten.

Die Ausgangsverbindung der Formel VI und deren Herstellung wird beispielsweise in der DE-OS Nr. 22 07 517 beschrieben.

Verbindungen der allgemeinen Formel VIII und IX können nach der im ersten Verfahren beschriebenen Methode aus den entsprechenden Ketocarbonsäuren durch Umsetzung mit Hydrazin erhalten werden.

Die Erfindung betrifft auch diejenigen Ausführungsformen eines Verfahrens, bei denen man ein Verfahren auf irgendeiner Stufe abbricht oder bei denen man von einer auf irgendeiner Stufe als Zwischenprodukt erhältlichen Verbindung ausgeht und die fehlenden Schritte durchführt, oder einen Ausgangsstoff unter den Reaktionsbedingungen bildet oder gegebenenfalls in Form eines Salzes verwendet. Die Erfindung beinhaltet auch daraus resultierende neue Zwischenprodukte.

Von der Erfindung ebenfalls umfasst sind therapeutische Stoffzusammenstellung, die aus einem antithrombotisch wirksamen Anteil der Verbindungen der Formel I und einem pharmakologisch annehmbaren festen Trägerstoff oder flüssigen Verdünnungsmittel bestehen.

Eine erhaltene Verbindung der Formel I, in der R eine Aminogruppe bedeutet kann gewünschtenfalls in ein Säureadditionssalz in an sich bekannter Weise überführt werden. Zur Herstellung von Säureadditionssalzen werden insbesondere solche Säuren verwendet, die zur Bildung von therapeutisch verwendbaren Salzen geeignet sind. Als solche Säuren seien beispielsweise genannt : Halogenwasserstoffsäuren, Schwefelsäuren, Phosphorsäuren, Salpetersäure, Perchlorsäure, aliphatische, alicyclische, aromatische oder heterocyclische Carbon- oder Sulfonsäuren wie Ameisen-, Essig-, Propion-, Bernstein-, Glykol-, Milch-, Aepfel-, Wein-, Zitronen-, Ascorbin-, Malein-, Hydroxymalein- oder Brenztraubensäure ; Phenylessig-, Benzoe-, p-Aminobenzoe-, Anthranil-, p-Hydroxybenzoe-, Salicyl- oder p-Aminosalicylsäure, Embonsäure, Methansulfon-, Aethansulfon-, Hydroxyäthansulfon-, Aethylensulfonsäure ; Halogenbenzolsulfon-, Toluolsulfon-, Naphthalinsulfonsäure oder Sulfanilsäure ; Methionin, Trypthophan, Lysin oder Arginin.

Je nach den Verfahrensbedingungen kann die Verbindung der Formel I, in der R eine Aminogruppe bedeutet auch als Säureadditionssalz anfallen.

Die Säureadditionssalze der neuen Verbindungen können in an sich bekannter Weise in die freie Verbindung übergeführt werden, z. B. mit basischen Mitteln, wie Alkalien oder Ionenaustauschern. Andererseits können die erhaltenen freien Basen mit organischen oder anorganischen Säuren Salze bilden.

Die erfindungsgemässen pharmazeutischen Präparate enthalten mindestens eine Verbindung der Formel I als Wirkstoff zusammen mit einem üblichen pharmazeutischen Trägerstoff. Die Art der Trägerstoffe richtet sich weitgehend nach dem Anwendungsgebiet. Die erfindungsgemässen pharmazeutischen Stoffzusammensetzungen, die als Wirkstoffe Verbindungen der Formel I enthalten, können oral, parenteral oder rektal verabreicht werden.

Zur oralen Behandlung von Thrombose kommen insbesondere feste Doseneinheitsformen, wie Tabletten, Dragées und Kapseln in Frage, die vorzugsweise zwischen 10 und 90 % eines Wirkstoffes der allgemeinen Formel I enthalten, um die Verabreichung von täglichen Dosen zwischen 1,0 bis 1 000 mg/kg, vorzugsweise zwischen 2 und 100 mg/kg, insbesondere zwischen 5 bis 10 mg/kg an Warmblütern von etwa 70 kg Körpergewicht zu ermöglichen. Zur Herstellung von Tabletten und Dragéekernen kombiniert man die Verbindungen der Formel I mit festen, pulverförmigen Trägerstoffen, wie Lactose, Saccharose, Sorbit, Maisstärke, Kartoffelstärke oder Amylopektin, Cellulosederivaten oder Gelatine, vorzugsweise unter Zusatz von Gleitmitteln, wie Magnesium- oder Calciumstearat, oder Polyäthylenglykolen von geeignetem Molekulargewicht. Dragéekerne überzieht man anschliessend beispielsweise mit konzentrierten Zuckerlösungen, welche z. B. noch arabischen Gummi, Talk und/oder Titandioxid enthalten können, oder mit einem in leichtflüchtigen organischen Lösungsmitteln oder Lösungsmittelgemischen gelösten Lack. Diesen Ueberzügen können Farbstoffe zugefügt werden, z. B. zur Kennzeichnung verschiedener Wirkstoffdosen. Weiche Gelatinekapseln und andere geschlossene Kapseln bestehen beispielsweise aus einem Gemisch von Gelatine und Glycerin und können z. B. Mischungen einer Verbindung der Formel I mit Polyäthylenglykol enthalten. Steckkapseln enthalten z. B. Granulate eines Wirkstoffes mit festen, pulverförmigen Trägerstoffen, wie z. B. Lactose, Saccharose, Sorbit, Mannit ; Stärken, wie Kartoffelstärke, Maisstärke oder Amylopektin, Cellulosederivate sowie Magnesiumstearat oder Stearinsäure.

Als Doseneinheitsformen für die rektale Anwendung kommen z. B. Suppositorien in Betracht, welche aus einer Kombination eines Wirkstoffes mit einer Suppositorien-Grundmase auf der Basis von natürlichen oder synthetischen Triglyceriden (z. B. Kakaobutter), Polyäthylenglykolen oder geeigneten höheren Fettalkoholen bestehen, und Gelatine-Rektalkapseln, welche eine Kombination des Wirkstoffes mit Polyäthylenglykolen enthalten.

Für Flüssigkeiten zur oralen Einnahme, wie Sirups und Elixiere, wird die Konzentration des Wirkstoffes derart gewählt, dass eine Einzeldosis leicht abgemessen werden kann, z. B. als Inhalt eines Teelöffels oder eines Messlöffels von z. B. 5 ml, oder auch als Mehrfaches dieser Volumina.

Die nachfolgenden Beispiele a) bis e) sollen die Herstellung einiger typischer Applikationsformer erläutern, jedoch keineswegs die einzigen Ausführungsformen von solchen darstellen.

a) 100,0 g Wirkstoff werden mit 610,0 g Lactose und 442,0 g Kartoffelstärke vermischt, die Mischung mit einer alkoholischen Lösung von 8 g Gelatine befeuchtet und durch ein Sieb granuliert. Nach dem Trocknen mischt man 60,0 g Talk, 10,0 g Magnesiumstearat und 20,0 g kolloidales Siliciumdioxid zu und presst die Mischung zu 10 000 Tabletten von je 125 mg Gewicht und 10 mg Wirkstoffgehalt, die gewünschtenfalls mit Teilkerben zur feineren Anpassung der Dosierung versehen sein können.

b) Aus 100,0 g Wirkstoff, 379,0 g Lactose und der alkoholischen Lösung von 6,0 g Gelatine stellt man ein Granulat her, das man nach dem Trocknen mit 10,0 g kolloidalem Siliciumdioxid, 40,0 g Talk, 60,0 g Kartoffelstärke und 5,0 g Magnesiumstearat mischt und zu 10 000 Dragéekernen presst. Diese werden anschliessend mit einem konzentrierten Sirup aus 533,5 g krist. Saccharose, 20,0 g Schellack, 75,0 g arabischem Gummi, 250,0 g Talk, 20,0 g kolloidalem Siliciumdioxid und 1,5 g Farbstoff überzogen und getrocknet. Die erhaltenen Dragées wiegen je 150 mg und enthalten je 10 mg Wirsktoff.

c) 10,0 g Wirkstoff und 1 990 g fein geriebene Suppositoriengrundmasse (z. B. Kakaobutter) werden gründlich gemischt und dann geschmolzen. Aus der durch Rühren homogen gehaltenen Schmelze werden 1 000 Suppositorien von 2,0 g gegossen. Sie Enthalten je 25 mg Wirkstoff.

d) Zur Bereitung eines Sirups mit 0,25 % Wirkstoffgehalt löst man in 3 Litern dest. Wasser 1,5 Liter Glycerin, 42 g p-Hydroxybenzoesäuremethylester, 18 g p-Hydroxybenzoesäure-n-propylester und unter leichtem Erwärmen 25,0 g Wirkstoff, fügt 4 Liter 70 %ige Sorbitlösung, 1 000 g krist. Saccharose, 350 g Glucose und einen Aromastoff, z. B. 250 g « Orange Peel Soluble Fluid » von Eli Lilly und Co., Indianapolis, oder 5 g natürliches Zitronenaroma und 5 g « Halb und Halb »-Essenz, beide von der Firma Haarmann und Reimer, Holzminden, Deutschland, zu, filtriert die erhaltene Lösung und ergänzt das Filtrat mit dest. Wasser auf 10 Liter.

e) Zur Bereitung einer Tropflösung mit 1,5 % Wirkstoffgehalt löst man 150,0 g Wirkstoff und 30 g Natriumcyclamat in einem Gemisch von 4 Liter Aethanol (96 %) und 1 Liter Propylenglykol. Andererseits mischt man 3,5 Liter 70 %ige Sorbitlösung mit 1 Liter Wasser und fügt die Mischung zur obigen Wirkstofflösung. Hierauf wird ein Aromastoff, z. B. 5 g Hustenbonbon-Aroma oder 30 g Grapefruit-Essenz, beide von der Firma Haarmann und Reimer, Holzminden, Deutschland zugegeben, das Ganze gut gemischt, filtriert und mit dest. Wasser auf 10 Liter ergänzt.

Die nachfolgenden Beispiele erläutern die Herstellung der neuen Verbindungen der Formel I, sollen jedoch den Umfang der Erfindung in keiner Weise beschränken. Die Temperaturen sind in Celsiusgraden angegeben.

## Beispiel 1

Zu einer Lösung von 10 g 3-Chlor-4-morpholino-benzoylpropionsäure in 120 ml Aethanol werden unter Rühren 2 g Hydrazinhydrat zugetropft. Das Reaktionsgemisch wird 2 Std. am Rückfluss gekocht, dann auf Raumtemperatur abgekühlt und abgenutscht. Das Nutschgut wird mit Aethanol nachgewaschen und am Hochvakuum bei 60° getrocknet. Man erhält so 9,2 g 6-(4-Morpholino-3-chlor-phenyl)-4,5-dihydro-3(2H)-pyridazinon vom Schmelzpunkt 187-189°.

## Beispiel 2

Zu einer Lösung von 3,7 g 6-(4-Morpholino-phenyl)-4,5-dihydro-3(2H)-pyridazinon in 5,7 ml Eisessig und 7,5 ml konzentrierter Salzsäure wird unter Rühren bei – 5 °C die Lösung von 0,65 g Natriumchlorat in 0,75 ml Wasser zugetropft. Das Reaktionsgemisch wird 30 Minuten bei 0 °C und danach 30 Minuten bei Raumtemperatur weitergerührt und danach auf 100 ml Eiswasser gegossen. Die Dabei entstehende Suspension wird mit konzentrierter Natronlauge auf pH 6 gestellt, mit Chloroform extrahiert, diese Chloroformextrakte mit Wasser nachgewaschen, über Natriumsulfat getrocknet und aus Aethanol umkristallisiert. Man erhält so das 6-(4-Morpholino-3-chlor-phenyl)-4,5-dihydro-3(2H)-pyridazinon vom Schmelzpunkt 184-186°.

## Beispiel 3

1,5 g 6-(3,4-Di-chlor-phenyl)-4,5-dihydro-3(2H)-pyridazinon werden zusammen mit 1,6 ml Morpholin 20 Std. am Rückfluss gekocht. Nach dem Abkühlen wird das Reaktionsgemisch zwischen Aethylacetat und 2-n-Salzsäure verteilt. Die organischen Phasen werden mit 5-n-Salzsäure extrahiert und aus diesen 5-n-Salzsäureextrakten erhält man nach Einstellen bis zur basischen Reaktion mit konz. Natronlauge, Zurückextrahieren mit Aethylacetat, Waschen mit Wasser und Sole, Trocknen und Eindampfen dieser Aethylacetatextrakte kristallines 6-(4-Morpholino-3-chlor-phenyl)-4,5-dihydro-3(2H)-pyridazinon. Schmelzpunkt nach Umkrist. aus Aethanol 184-186°.

## Beispiel 4

Zu einer Lösung von 1,5 g 6-(4-Morpholino-phenyl)-4,5-dihydro-3(2H)-pyridazinon in 11 ml Chloroform wird bei 0° unter Rühren die Lösung von 0,3 ml Brom in 4,5 ml Chloroform zugetropft. Die Reaktionslösung wird 30 Minuten bei 0° und weitere 60 Minuten bei Raumtemperatur weitergerührt und dann im Scheidetrichter mit 1-n Natriumbikarbonatlösung geschüttelt bis zwei klare Phasen entstehen. Die organische Phase wird mit Wasser gewaschen, getrockent über Natriumsulfat und eingedampft. Man erhält 1,9 g rohes 6-(4-Morpholino-3-brom-phenyl)-4,5-dihydro-3(2H)-pyridazinon. Dieses wird zur weiteren Reinigung unter Zugabe von wenig Methylenchlorid in 100 ml Aethylacetat gelöst. Die Lösung wird dreimal mit 2-n-Salzsäure gewaschen und dann mit 5-n-Salzsäure extrahiert. Aus diesen Extrakten wird nach basisch stellen mit Natronlauge, Extrahieren mit Aethylacetat-Methylenchlorid, Waschen, Trocknen und Eindampfen der organischen Phasen 1,3 g gereinigtes Produkt erhalten, welches nach Umkristallisieren aus Aethanol bei 175-176° schmilzt.

## Beispiel 5

170 g 3-Chlor-4-morpholino-benzoyl-propionsäure-morpholinamid werden zusammen mit 68 ml Hydrazinhydrat in 2,5 ltr. 50 %-iger Essigsäure 2 Stunden unter Erhitzen zum Rückfluss und anschliessend 16 Stunden bei Raumtemperatur gerührt. Das ausgefallene Produkt wird abgenutscht, mit Wasser gewaschen und unter Hochvakuum bei 80° getrocknet. Man erhält so 126 g kristallines 6-(3-Chlor-4-morpholinophneyl)-4,5-dihydro-3(2H)-pyridazinon. Dieses wird in 750 ml Dimethylformamid unter Erwärmen gelöst, abgekühlt, mit wässrigem Aethanol (50 %) versetzt, abgenutscht und unter Hochvakuum bei 60° getrocknet. Smp. 185-189°.

Das als Ausgangsmaterial benötigte 3-Chlor-4-morpholino-benzoyl-propionsäure-morpholinamid wird wie folgt hergestellt :

200 g 3,4-Dichlor-benzoyl-propionsäure werden zusammen mit 350 ml Morpholin 24 Std. unter Erhitzen zum Rückfluss gerührt. Das Reaktionsgemisch wird abgekühlt und zwischen Essigester und 2-n Salzsäure verteilt. Die Essigesterphasen werden 6 mal mit 500 ml 5-n Salzsäure extrahiert. Diese gesammelten Salzsäureextrakte werden mit konzentrierter Natronlauge auf pH 5-6 gestellt und die dabei ausfallenden Kristalle werden abgenutscht und bei 50° unter Hochvakuum getrocknet. Man erhält so 198 g 3-Chlor-4-morpholino-benzoyl-propionsäure-morpholinamid vom Smp. 94-102°. Der Schmelzpunkt erhöht sich nach Umkristallisieren aus Aethylacetat-Petroläther auf 103-106°.

## Beispiel 6

1 g 6-(3-Amino-4-morpholino-phenyl)-4,5-dihydro-3(2H)-pyridazinon werden mit 9 ml Wasser und 9 ml konz. Salzsäure versetzt und bei 0-5° mit einer Lösung von 0,29 g Natriumnitrit in 1 ml Wasser diazotiert. Nach 15 Minuten wird überschüssiges Nitrit in der Lösung durch Zugabe von Harnstoff vernichtet und das Reaktionsgemisch wird mit einer Lösung von 1,8 g Kupfer (I)-chlorid in 9 ml Wasser und 9 ml konz. Salzsäure versetzt. Nun wird die Reaktionstemperatur von 0-5° auf 25° gebracht und noch 3 Stunden unter Rühren bei 25° belassen. Das Reaktionsgemisch wird noch 2 Stunden bei 40° gerührt, dann schwach alkalisch auf pH 10 gestellt, mit Essigester versetzt und durch Hyflo filtriert. Die organische Phase wird abgetrennt und die wässrige Phase des Filtrates wird noch zweimal mit Essigester extrahiet. Alle gesammelten organischen Phasen werden vereinigt, über Natriumsulfat getrocknet und eingedampft. Das als Rückstand erhaltene 6-(4-Morpholino-3-chlor-phenyl)-4,5-dihydro-3(2H)-pyridazinon kristallisiert spontan mit einem Schmelzpunkt von 188-192°.

Das als Ausgangsmaterial verwendete 6-(3-Amino-4-morpholino-phenyl)-4,5-dihydro-3(2H)-pyridazinon wird wie folgt erhalten :

90 g 6-(4-Morpholino-3-nitrophenyl)-4,5-dihydro-3(2H)-pyridazinon (J. Het. Chem. *15*, 881 (1978)) werden in 2,7 ltr. Aethanol mit 9 g 5 %-igem Palladium auf Kohle Katalysator bei Raumtemperatur und Normaldruck hydriert. Das Reaktionsgemisch wird filtriert und das Aethanol-Filtrat eingedampft. Der Katalysator wird in 1-n Salzsäure aufgeschlämmt, abfiltriert und mit 1-n Salzsäure nachgewaschen. Die wässrigen Phasen werden mit konz. Natronlauge auf pH 6 gestellt und das ausgefallene Produkt wird abgenutscht und mit Wasser nachgewaschen. Das so erhaltene Produkt wird mit dem Eindampfrückstand des Aethanolfiltrates vereinigt und in Dimethylformamid unter Erwärmen gelöst, mit Aktivkohle behandelt, über Hyflo filtriert und aus dem Filtrat wird durch Kühlen und versetzen mit Wasser das 6-(3-Amino-4-morpholino-phenyl)-4,5-dihydro-3(2H)-pyridazinon auskristallisiert, welches nach Trocknen unter Hochvakuum bei 100° bei 249-253° schmilzt.

## Beispiel 7

1 g 6-(3-Chlor-4-amino-phenyl)-4,5-dihydro-3(2H)-pyridazinon werden zusammen mit 1,7 g 2,2'-Dibrom-diäthyläther, 2,5 ml Diisopropyläthylamin und 10 ml Dimethylformamid 18 Std. unter Erhitzen bei 100° gerührt. Das Reaktionsgemisch wird abgekühlt und zwischen Essigester und 5-n Salzsäure verteilt. Die Salzsäureextrakte werden mit Natronlauge auf pH 6 gestellt und mit Methylenchlorid extrahiert. Die

gesammelten organischen Phasen werden mit Wasser gewaschen, über Natriumsulfat getrocknet und eingedampft. Man erhält 1 g rohes 6-(4-Morpholino-3-chlor-phenyl)-4,5-dihydro-3(2H)-pyridazinon, welches nach Umkristallisation aus Aethanol bei 183-185° schmilzt.

Das als Ausgangsmaterial benötigte 6-(3-Chlor-4-amino-phenyl)-4,5-dihydro-3(2H)-pyridazinon wird wie folgt hergestellt :

4-Acetylamino-benzoylpropionsäure, erhalten durch Friedel-Crafts Acylierung von Acetanilid mit Bernsteinsäureanhydrid, wird analog wie im Beispiel 2 beschrieben in die 3-Chlor-4-acetylamino-benzoylpropionsäure übergeführt. Diese wird durch Hydrolyse in 18 %-iger Salzsäure in die 3-Chlor-4-amino-benzoylpropionsäure übergeführt (Smp. ungereinigt 156-158°). Daraus erhält man analog Beispiel 1 durch Umsetzen mit Hydrazin das 6-(3-Chlor-4-amino-phenyl)-4,5-dihydro-3(2H)-pyridazinon (Smp. ungereinigt 225-230°).

## Beispiel 8

Analog Beispiel 1 werden 7 g 3-Fluor-4-morpholinobenzoylpropionsäure zusammen mit 1,32 ml Hydrazinhydrat und 90 ml Aethanol 2 Stunden zum Rückfluss erhitzt. Das Reaktionsgemisch wird auf 0° abgekühlt, eine Stunde bei dieser Temperatur weitergerührt und die ausgefallenen Kristalle werden abgenutscht und mit geringen Mengen Aethanol nachgewaschen. Durch Trocknen der Kristalle unter Hochvakuum bei 50° erhält man 6,25 g 6-(3-Fluorphenyl-4-morpholino)-4,5-dihydro-3(2H)-pyridazinon mit einem Schmelzpunkt von 167-170°.

Die als Ausgangsmaterial verwendete 3-Fluor-4-morpholino-benzoylpropionsäure wird wie folgt hergestellt :

33 g 3,4-Di-fluor-benzoylpropionsäure (erhalten durch Friedel-Crafts Acylierung von 1,2-Di-fluorbenzol mit Bernsteinsäureanhydrid in Gegenwart von Aluminiumchlorid) werden zusammen mit 60 ml Morpholin 20 Stunden unter Rühren auf 150° erhitzt. Das Reaktionsgemisch wird abgekühlt, in Aethylacetat gelöst und diese Lösung wird mit 2-n Salzsäure gewaschen und mit 5-n Salzsäure extrahiert. Die 5-n Salzsäureextrakte werden mit konzentrierter Natronlauge auf 6 pH gestellt und die dabei entstehende Suspension wird mit Aethylacetat extrahiert. Die organischen Phasen werden mit Wasser gewaschen, über Natriumsulfat getrocknet und eingedampft. Der kristalline Rückstand (31,7 g) wird zusammen mit 18 g Kaliumhydroxid, 180 ml Wasser und 180 ml Aethanol 10 Stunden zum Rückfluss erhitzt, abgekühlt, eingedampft und zwischen Wasser und Aethylacetat verteilt. Die wässrigen Phasen werden auf pH 5 gestellt, die dabei ausgefallene 3-Fluor-4-morpholino-benzoylpropionsäure wird abgenutscht und getrocknet (Smp. 164-168°).

## Beispiel 9

5,8 g 4-Morpholino-3-trifluormethyl-benzoyl-propionsäure-morpholinamid werden zusammen mit 2,2 ml Hydrazinhydrat und 90 ml 50 %-iger Essigsäure unter Rühren 1 Stunde zum Rückfluss erhitzt. Das Reaktionsgemisch wird abgekühlt, am Rotationsverdampfer eingedampft, mit wenig Wasser versetzt, mit 2-n Sodalösung auf pH 6 gestellt und mit Methylenchlorid extrahiert. Die Methylenchlorid-extrakte werden mit Wasser gewaschen, über Natriumsulfat getrocknet und eingedampft. Aus dem Rückstand erhält man durch Umkristallisieren aus Aethanol-Petroläther das 6-(3-Trifluormethyl-4-morpholino-phenyl)-4,5-dihydro-3(2H)-pyridazinon vom Schmelzpunkt von 163-166°.

Das als Ausgangsmaterial benötigte 4-Morpholino-3-triluromethyl-benzoylpropionsäure-morpholinamid wird wie folgt hergestellt :

12,3 g o-(4-Chlor-3-trifluormethyl-phenyl)-4-morpholino-acetonitril (J. Het. Chem. *15*, 883 (1978)) werden in 150 ml Tetrahydrofuran gelöst und bei 5° unter Rühren mit 5 ml 30 %-iger äthanolischer Kaliumhydroxidlösung versetzt. Das Reaktionsgemisch wird 15 Minuten bei 5° weitergerührt und dann unter den gleichen Bedingungen tropfenweise mit der Lösung von 5,9 g Acrylsäureäthylester in 100 ml Tetrahydrofuran versetzt. Das Reaktionsgemisch wird 1 Stunde bei Raumtemperatur weitergerührt und dann am Rotationsverdampfer eingeengt. Zum erhaltenen Rückstand wird dreimal Toluol zugegeben und dieses am Rotationsverdampfer wieder abgedampft. Der so erhaltene Rückstand wird in Aether aufgenommen und klar filtriert. Durch Eindampfen des Filtrates erhält man 14,3 g rohes γ-Cyano-γ-(4-chlor-3-trifluormethyl-phenyl)-γ-(4-morpholino)-buttersäureäthylester, welcher in zwei Schritten wie folgt verseift wird :

12,3 g γ-Cyano-γ-(4-chlor-3-trifluormethyl-phenyl)-γ-(4-morpholino)-buttersäureäthylester werden zusammen mit 75 ml Essigsäure und 32 ml Wasser 2 Stunden unter Erhitzen auf 100° gerührt. Das Reaktionsgemisch wird am Rotationsverdampfer eingedampft eingedampft und der Rückstand wird in 250 ml Aethanol und 9,2 ml Wasser gelöst. Das Reaktionsgemisch wird mit festen Kaliumhydroxid alkalisch gestellt, mit weiteren 1,9 g festem Kaliumhydroxid versetzt und 30 Minuten unter Erhitzen zum Rückfluss und 16 Stunden bei Raumtemperatur gerührt.

Das Reaktionsgemisch wird eingedampft, der Rückstand wird zwischen Essigester und Wasser verteilt, die wässrigen Phasen werden mit konz. Salzsäure sauer gestellt und mit Aethylacetat extrahiert. Aus diesem Extrakt erhält man durch Eindampfen 6,2 g 4-Chlor-3-trifluormethyl-benzoylpropionsäure vom Schmelzpunkt 87-96°. Das ungereinigte Material wird wie folgt direkt weiter verwendet.

3 g 4-Chlor-3-trifluormethyl-benzoyl-propionsäure werden zusammen mit 6,5 ml Morpholin 20 Stunden unter Erhitzen zum Rückfluss gerührt. Das Reaktionsgemisch wird abgekühlt, in Essigsäureäthylester aufgenommen und mit 1-n Salzsäure und Wasser gewaschen, über Natriumsulfat getrocknet und eingedampft. Als Rückstand erhält man 3,2 g ungereinigtes 4-Morpholino-3-trifluormethyl-benzoyl-propionsäure-morpholinamid welches direkt weiterverwendet werden kann. Nach Umkristallisieren aus einem Gemisch aus Aether/Petroläther erhält man Kristalle vom Schmelzpunkt 76-79°.

Beispiel 10

Zu einer Suspension von 10 g 6-(3-Amino-4-morpholino-phenyl)-4,5-dihydro-3(2H)-pyridazinon (erhalten nach Beispiel 6, 6,2 ml Di-isopropyläthylamin und 120 ml Dimethylformamid wird die Lösung von 3,5 ml Essigsäureanhydrid in 30 ml Dimethylformamid zugetropft. Das Reaktionsgemisch wird 48 Stunden bei Raumptemperatur weitergerührt und dann am Rotationsverdampfer eingedampft. Der Rückstand wird zwischen Aethylacetat und 2-n Salzäure verteilt. Die Salzsäureextrakte werden vereinigt und mit konzentrierter Natronlauge basisch gestellt. Das dabei ausgefallene Produkt wird abgenutscht, mit Wasser gewaschen und am Hochvakuum getrocknet. Durch Umkristallisieren aus Dimethylformamid-Aethanol und Reinigen über Aktivkohle erhält man 7,5 g 6-(3-Acetamino-4-morpholino-phenyl)-4,5-dihydro-3(2H)-pyridazinon vom Schmelzpunkt von 240-242°.

Beispiel 11

2,2 g 6-(4-Amino-3-methyl-phenyl)-4,5-dihydro-3(2H)-pyridazinon werden zusammen mit 5,5 ml Diisopropyläthylamin, 3,7 g Dibromdiäthyläther und 11 ml Dimethylformamid 6 Stunden lang bei 100° gerührt. Das Reaktionsgemisch wird auf Raumtemperatur abgekühlt und mit Essigester versetzt. Das dabei ausgefallene Diisopropylaminhydrobromid wird abgenutscht und mit Essigester nachgewaschen. Die vereinigten Filtrate werden mit Wasser gewaschen, über Natriumsulfat getrocknet und eingedampft. Der Rückstand wird über Silicagel chromatographiert. Nach dem Abtrennen von unpolaren Verunreinigungen mit einem Gemisch von Toluol : Essigester = 1 : 1 wird das 6-(4-Morpholino-3-methyl-phenyl)-4,5-dihydro-3(2H)-pyridazinon mit Essigester eluiert und schmilzt nach Umkristallisieren aus einem Gemisch von Methylenchlorid/Petroläther bei 208-211°.

Das als Ausgangsmaterial benötigte 6-(4-Amino-3-methyl-phenyl)-4,5-dihydro-3(2H)-pyridazinon wird wie folgt hergestellt :

Aus 70 g N-Acetyl-1,2-toluidin wird mit 47 g Bernsteinsäureanhydrid und 234 g Aluminiumchlorid in 2 000 ml Tetrachloräthan nach der üblichen Methode 50 g 3-(3-Methyl-4-acetamido-benzoyl)-propionsäure vom Schmelzpunkt von 150-160° als Rohprodukt erhalten. Daraus erhält man durch Hydrolyse in 18 %-iger Salzsäure 28 g rohe 3-(4-Amino-3-methyl-benzoyl)-propionsäure. Aus 6 g 3-(4-Amino-3-methyl-benzoyl)-propionsäure erhält man mit 1,7 ml Hydrazinhydrat in 70 ml Aethanol analog Beispiel 1) 2,6 g 6-(4-Amino-3-methyl-phenyl)-4,5-dihydro-3(2H)-pyridazinon vom Schmelzpunkt 215-225°.

Beispiel 12

25 g 3-Amino-4-morpholino-benzoylpropionsäure werden in 24,2 g konzentrierter Salzsäure und 70 ml Wasser gelöst und bei 0° mit einer Lösung von 7,7 g Natriumnitrit in 40 ml Wasser diazotiert. Nun wird ein Reagens (hergestellt durch Vermischen einer Lösung von 27 g Kaliumcyanid in 50 ml Wasser mit der auf 60° aufgewärmten Lösung von 24,2 g Kupfersulfat-pentahydrat in 95 ml Wasser) auf Raumtemperatur abgekühlt und bei 0-5° zur Diazoniumlösung hinzugegeben. Das Reaktionsgemisch wird danach 16 Stunden bei Raumtemperatur weitergerührt, mit Wasser verdünnt, mit konzentrierter Natronlauge auf pH 14 gestellt, und mit Essigester dreimal gewaschen. Die wässrigen Phasen werden mit Aktivkohle gereinigt, mit konzentrierter Salzsäure auf pH 2 gestellt und mit Essigester extrahiert. Die organischen Extrakte werden klar filtriert, mit 2-n Salzsäure und mit Wasser gewaschen, über Natriumsulfat getrocknet und eingedampft. Man erhält 16 g rohe 3-Cyan-4-morpholino-benzoyl-propionsäure, welche mit Hydrazinhydrat analog Beispiel 1 umgesetzt wird. Man erhält das 6-(3-Cyan-4-morpholino-phenyl)-4,5-dihydro-3(2H)-pyridazinon vom Schmelzpunkt von 212-214°, umkristallisiert aus Methylenchlorid-Dimethylformamid.

Die als Ausgangsmaterial benötigte 3-Amino-4-morpholino-benzoyl-propionsäure wird durch Umsatz der 3-Nitro-4-chlorbenzoylpropionsäure mit Morpholin analog Beispiel 8 und Reduktion der dabei entstehenden 3-Nitro-4-morpholino-benzoylpropionsäure erhalten.

**Patentansprüche** (für die Vertragsstaaten : BE, CH, DE, FR, IT, LI, LU, NL, SE)

1. Pyridazinone der allgemeinen Formel I

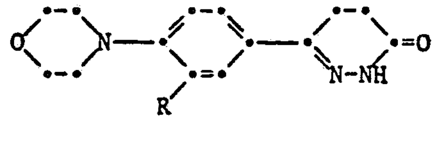

(I)

worin R ein Halogenatom, die Amino-, Acetylamino-, Methyl-, Cyano-, Methoxy- oder die Trifluormethylgruppe bedeutet, und ihre tautomeren Formen.

2. Pyridazinone der allgemeinen Formel I

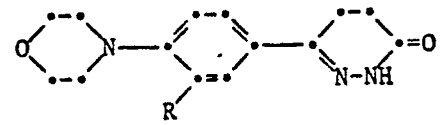

(I)

worin R ein Halogenatom oder die Trifluormethylgruppe bedeutet, und ihre tautomeren Formen.

3. Pyridazinone der allgemeinen Formel I gemäss Anspruch 1, worin R ein Chlor- oder auch Bromatom bedeutet, und ihre tautomeren Formen.

4. 6-(3-Chlor-4-morpholino-phenyl)-4,5-dihydro-3(2H)-pyridazinon und seine tautomeren Formen.

5. 6-(3-Amino-4-morpholino-phenyl)-4,5-dihydro-3(2H)-pyridazinon und seine tautomeren Formen.

6. 6-(3-Acetylamino-4-morpholino-phenyl)-4,5-dihydro-3(2H)-pyridazinon und seine tautomeren Formen.

7. 6-(3-Cyano-4-morpholino-phenyl)-4,5-dihydro-3(2H)-pyridazinon und seine tautomeren Formen.

8. Pharmazeutische Präparate enthaltend eine der Verbindungen des Anspruchs 1.

9. Die Verbindungen des Anspruchs 1 zur Anwendung in einem Verfahren zur therapeutischen Behandlung des menschlichen oder tierischen Körpers.

10. Die Verbindungen des Anspruchs 1 zur Verwendung als antithrombotisch wirksames Mittel.

11. Verwendung von Verbindungen des Anspruchs 1 zur Herstellung von pharmazeutischen Präparaten.

12. Pharmazeutische Präparate enthaltend eine der Verbindungen der Ansprüche 2-4.

13. Die Verbindungen der Ansprüche 2-4 zur Anwendung in einem Verfahren zur therapeutischen Behandlung des menschlichen oder tierischen Körpers.

14. Die Verbindungen der Ansprüche 2-4 zur Verwendung als antithrombotisch wirksames Mittel.

15. Verwendung von Verbindungen der Ansprüche 2-4 zur Herstellung von pharmazeutischen Präparaten.

16. Verfahren zur Herstellung von Verbindungen der allgemeinen Formel I

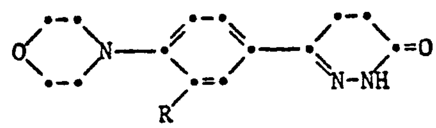

(I)

worin R ein Halogenatom, die Amino-, Acetylamino-, Methyl-, Cyano-, Methoxy- oder die Trifluormethylgruppe bedeutet, und ihre tautomeren Formen, dadurch gekennzeichnet, dass man

a) eine Ketocarbonsäure der Formel II

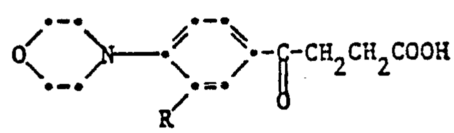

(II)

oder ein reaktionsfähiges Derivat einer solchen Ketocarbonsäure, worin R die unter der Formel I angegebene Bedeutung hat, mit Hydrazin umsetzt, oder

b) eine Verbindung der Formel III

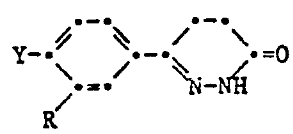

(III)

worin R die unter der Formel I angegebene Bedeutung hat, und Y eine zusammen mit Wasserstoff abspaltbare Gruppe bedeutet, mit Morpholin umsetzt, oder

c) eine Verbindung der Formel IV

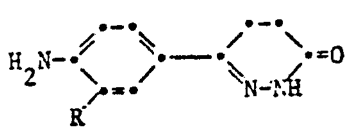

(IV)

mit einem Diäthylätherderivat der Formel V

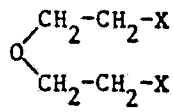 (V)

worin X eine zusammen mit Wasserstoff abspaltbare Gruppe bedeutet, umsetzt, und/oder, wenn erwünscht, eine erhaltene Verbindung der Formel I, in der R eine Aminogruppe bedeutet, diese in eine Acetylaminogruppe überführt, und/oder, wenn erwünscht, eine erhaltene Verbindung der Formel I, in der R eine Aminogruppe bedeutet, in ein Säureadditionssalz überführt, und/oder, wenn erwünscht, erhaltene Säureadditionssalze der Verbindung der Formel I in eine Verbindung mit freier Aminogruppe überführt.

17. Verfahren zur Herstellung von Verbindungen der im Anspruch 1 gezeigten Formel I, in der R ein Halogenatom oder eine Cyanogruppe bedeutet, dadurch gekennzeichnet, dass man

a) eine Verbindung der Formel VI

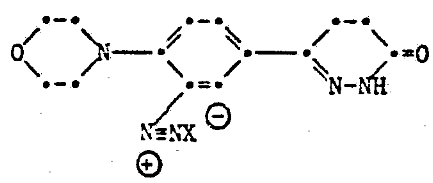 (VI)

worin $X^{\ominus}$ ein Anion einer Mineralsäure bedeutet, in Gegenwart von Kupfer oder eines Kupfer-I-salzes ein Halogenatom oder eine Cyanogruppe als R einführt, oder

b) eine Verbindung der Formel VII

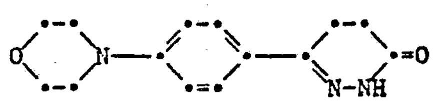 (VII)

halogeniert.

18. Verfahren zur Herstellung von Verbindungen der im Anspruch 1 gezeigten Formel I, in der R eine Aminogruppe bedeutet, dadurch gekennzeichnet, dass man

a) eine Verbindung der Formel VIII

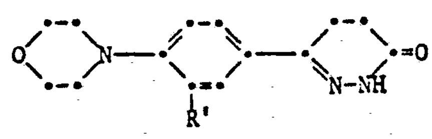 (VIII)

worin R' eine durch eine Schutzgruppe geschützte Aminogruppe bedeutet, solvolysiert, oder

b) in einer Verbindung der Formel

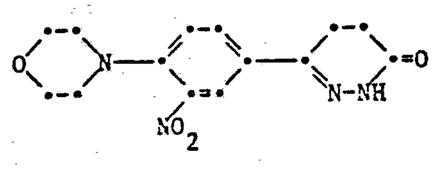 (IX)

die Nitrogruppe zur Aminogruppe selektiv reduziert, und/oder, wenn erwünscht eine erhaltene Verbindung der Formel I, in der R eine Aminogruppe bedeutet, diese in eine Acetylaminogruppe überführt, und/oder, wenn erwünscht, eine erhaltene Verbindung der Formel I, in der R eine Aminogruppe bedeutet, in ein Säureadditionssalz überführt, und/oder, wenn erwünscht, erhaltene Säureadditionssalze der Verbindungen der Formel I in eine Verbindung mit freier Aminogruppe überführt.

19. Die nach den Verfahren gemäss den Ansprüchen 16, 17 und 18 erhältlichen Verbindungen.

**Patentansprüche** (für den Vertragsstaat AT)

1. Verfahren zur Herstellung von Verbindungen der allgemeinen Formel I

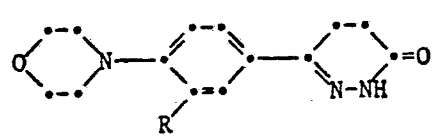 (I)

worin R ein Halogenatom, die Amino-, Acetylamino-, Methyl-, Cyano-, Methoxy- oder die Trifluormethylgruppe bedeutet, und ihre tautomeren Formen, dadurch gekennzeichnet, dass man

a) eine Ketocarbonsäure der Formel II

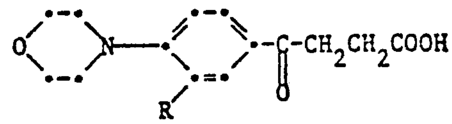

(II)

oder ein reaktionsfähiges Derivat einer solchen Ketocarbonsäure, worin R die unter der Formel I angegebene Bedeutung hat, mit Hydrazin umsetzt, oder

b) eine Verbindung der Formel III

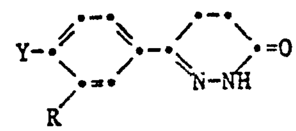

(III)

worin R die unter der Formel I angegebene Bedeutung hat, und Y eine zusammen mit Wasserstoff abspaltbare Gruppe bedeutet, mit Morpholin umsetzt, oder

c) eine Verbindung der Formel IV

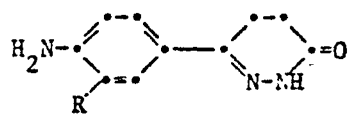

(IV)

mit einem Diäthylätherderivat der Formel V

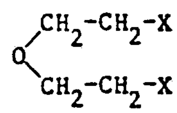

(V)

worin X eine zusammen mit Wasserstoff abspaltbare Gruppe bedeutet, umsetzt, und/oder, wenn erwünscht, eine erhaltene Verbindung der Formel I, in der R eine Aminogruppe bedeutet, diese in eine Acetylaminogruppe überführt, und/oder, wenn erwünscht, eine erhaltene Verbindung der Formel I, in der R eine Aminogruppe bedeutet, in ein Säureadditionssalz überführt, und/oder, wenn erwünscht, erhaltene Säureadditionssalze der Verbindung der Formel I in eine Verbindung mit freier Aminogruppe überführt.

2. Verfahren zur Herstellung von Verbindungen der im Anspruch 1 gezeigten Formel I, in der R ein Halogenatom oder die Cyanogruppe bedeutet, dadurch gekennzeichnet, dass man

a) in einer Verbindung der Formel VI

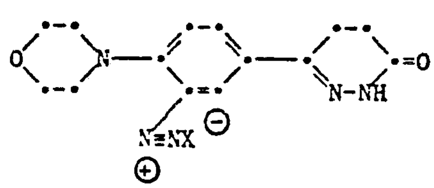

(VI)

worin $X^{\ominus}$ ein Anion einer Mineralsäure bedeutet, in Gegenwart von Kupfer oder eines Kupfer-I-salzes ein Halogenatom oder eine Cyanogruppe als R einführt, oder

b) eine Verbindung der Formel VII

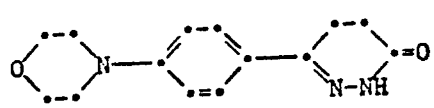

(VII)

halogeniert.

3. Verfahren zur Herstellung von Verbindungen der im Anspruch 1 gezeigten Formel I, in der R eine Aminogruppe bedeutet, dadurch gekennzeichnet, dass man

a) eine Verbindung der Formel VIII

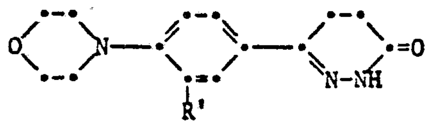

(VIII)

worin R' eine durch eine Schutzgruppe geschützte Aminogruppe bedeutet, solvolisiert, oder

b) in einer Verbindung der Formel

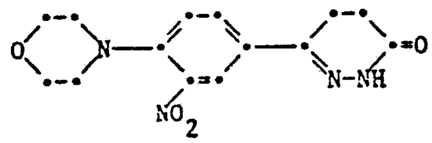

(IX)

die Nitrogruppe zur Aminogruppe selektiv reduziert, und/oder, wenn erwünscht eine erhaltene Verbindung der Formel I, in der R eine Aminogruppe bedeutet, diese in eine Acetylaminogruppe überführt, und/oder, wenn erwünscht, eine erhaltene Verbindung der Formel I, in der R eine Aminogruppe bedeutet, in ein Säureadditionssalz überführt, und/oder, wenn erwünscht, erhaltene Säureadditionssalze der Verbindungen der Formel I in eine Verbindung mit freier Aminogruppe überführt.

4. Verfahren zur Herstellung von Verbindungen der allgemeinen Formel I

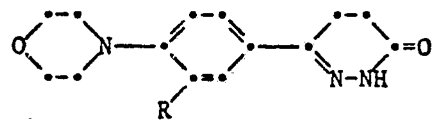

(I)

worin R ein Halogenatom oder die Trifluormethylgruppe bedeutet, und ihre tautomeren Formen, dadurch gekennzeichnet, dass man

a) eine Ketocarbonsäure der Formel II

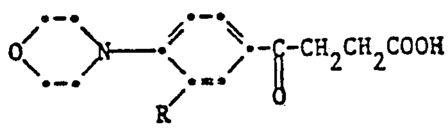

(II)

oder ein reaktionsfähiges Derivat einer solchen Ketocarbonsäure, worin R die unter der Formel I angegebene Bedeutung hat, mit Hydrazin umsetzt,

b) eine Verbindung der Formel III

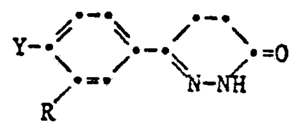

(III)

worin R die unter der Formel I angegebene Bedeutung hat, und Y eine zusammen mit Wasserstoff abspaltbare Gruppe bedeutet, mit Morpholin umsetzt.

5. Verfahren zur Herstellung von Verbindungen der im Anspruch 4 gezeigten Formel I, dadurch gekennzeichnet, dass man eine Verbindung der Formel VII

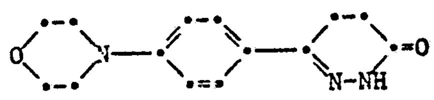

(VII)

halogeniert.

6. Verfahren gemäss Anspruch 5 zur Herstellung von Verbindungen der Formel I, dadurch gekennzeichnet, dass man die Halogenierung einer Verbindung der Formel VII durch Erhitzen einer Verbindung der Formel VI

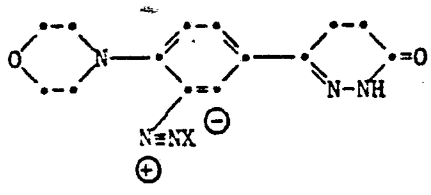

(VI)

worin $X^{\ominus}$ ein Anion einer Mineralsäure bedeutet, ausführt.

7. Verfahren gemäss den Ansprüchen 5 und 6 zur Herstellung von Verbindungen der Formel I, dadurch gekennzeichnet, dass man die Halogenierung durch Erhitzen einer Verbindung der Formel VI in Gegenwart von Kupferpulver ausführt.

8. Verfahren nach Patentanspruch 1, dadurch gekennzeichnet, dass man eine Verbindung der Formel I und ihre tautomeren Formen herstellt, in der R ein Halogenatom, die Amino-, Acetylamino-, Methyl-, Cyano-, Methoxy-, oder die Trifluormethylgruppe bedeutet.

9. Verfahren nach Patentanspruch 4, dadurch gekennzeichnet, dass man eine Verbindung der Formel I und ihre tautomeren Formen herstellt, in der R ein Halogenatom bedeutet.

10. Verfahren nach Patentanspruch 4, dadurch gekennzeichnet, dass man eine Verbindung der Formel I und ihre tautomeren Formen herstellt, in der R ein Chloratom bedeutet.

11. Verfahren nach Patentanspruch 1, dadurch gekennzeichnet, dass man eine Verbindung der Formel I und ihre tautomeren Formen herstellt, in der R eine Aminogruppe bedeutet.

12. Verfahren nach Patentanspruch 1, dadurch gekennzeichnet, dass man eine Verbindung der Formel I und ihre tautomeren Formen herstellt, in der R eine Acetylaminogruppe bedeutet.

13. Verfahren nach Patentanspruch 1, dadurch gekennzeichnet, dass man eine Verbindung der Formel I und ihre tautomeren Formen herstellt, in der R eine Cyanogruppe bedeutet.

14. Pharmazeutische Präparate enthaltend eine der Verbindungen, die gemäss den Ansprüchen 1, 2, 3, 8 und 11-13 hergestellt werden.

15. Pharmazeutische Präparate enthaltend eine der Verbindungen, die gemäss den Ansprüchen 4-7, 9 und 12 hergestellt werden.

**Claims** (for the Contracting States : BE, CH, DE, FR, IT, LI, LU, NL, SE)

1. A pyridazinone of the general formula I

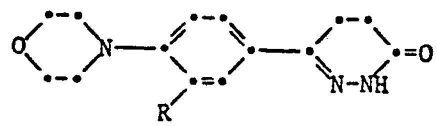

(I)

wherein R is a halogen atom, or the amino, acetylamino, methyl, cyano, methoxy or trifluoromethyl group, or a tautomeric form thereof.

2. A pyridazinone of the general formula I

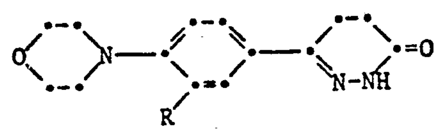

(I)

wherein R is a halogen atom or the trifluoromethyl group, or a tautomeric form thereof.

3. A pyridazinone of the general formula I according to claim 1, wherein R is a chlorine or bromine atom, or a tautomeric form thereof.

4. 6-(3-Chloro-4-morpholinophenyl)-4,5-dihydro-3(2H)-pyridazinone, or a tautomeric form thereof.

5. 6-(3-Amino-4-morpholinophenyl)-4,5-dihydro-3(2H)-pyridazinone, or a tautomeric form thereof.

6. 6-(3-Acetylamino-4-morpholinophenyl)-4,5-dihydro-3(2H)-pyridazinone, or a tautomeric form thereof.

7. 6-(3-Cyano-4-morpholinophenyl)-4,5-dihydro-3(2H)-pyridazinone, or a tautomeric form thereof.

8. A pharmaceutical composition containing a compound according to claim 1.

9. A compound according to claim 1 for application in a process for the therapeutic treatment of the human or animal body.

10. A compound according to claim 1 for use as an antithrombotically effective agent.

11. Use of a compound according to claim 1 for the preparation of a pharmaceutical composition.

12. A pharmaceutical composition containing a compound according to any one of claims 2 to 4.

13. A compound according to any one of claims 2 to 4 for application in a process for the therapeutic treatment of the human or animal body.

14. A compound according to any one of claims 2 to 4 for use as an antithrombotically effective agent.

15. Use of a compound according to any one of claims 2 to 4 for the preparation of a pharmaceutical composition.

16. A process for the preparation of a compound of the general formula I

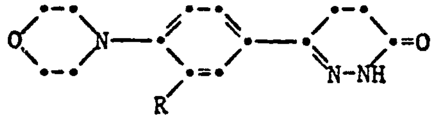

(I)

wherein R is a halogen atom, or the amino, acetylamino, methyl, cyano, methoxy or trifluoromethyl group, which processus comprises

a) reacting a ketocarboxylic acid of formula II

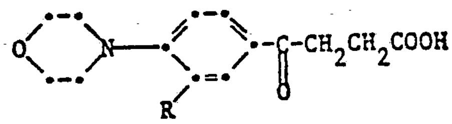

(II)

or a reactive derivative of such a ketocarboxylic acid, wherein R is as defined formula I, with hydrazine ; or

b) reacting a compound of formula III

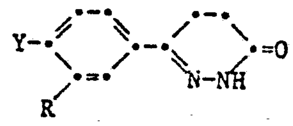

(III)

wherein R is as defined for formula I, and Y is a group which is detachable together with hydrogen, with morpholine ; or

c) reacting a compound of formula IV

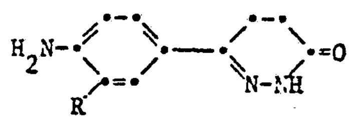

(IV)

with a diethyl ether derivative of formula V

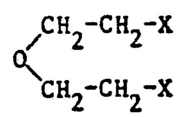

(V)

wherein X is a group which is detachable together with hydrogen ; and/or, if desired, converting in a resultant compound of formula I, in which R is an amino group, said group into an acetylamino group, and/or, if desired, converting a resultant compound of formula I, in which R is an amino group, into an acid addition salt, and/or, if desired, converting a resultant acid addition salt of a compound of formula I into a compound containing a free amino group ; or of a tautomeric form thereof.

17. A process for the preparation of a compound of formula I according to claim 1, wherein R is a halogen atom or a cyano group, which process comprises

a) introducing into a compound of formula VI

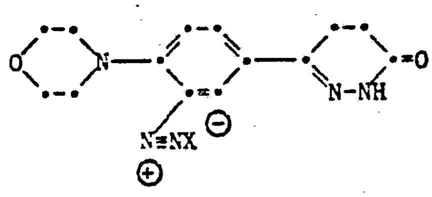

(VI)

wherein $X^{\ominus}$ is an anion of a mineral acid, in the presence of copper or of a copper (I) salt, a halogen atom or a cyano group as R ; or

b) halogenating a compound of formula VII

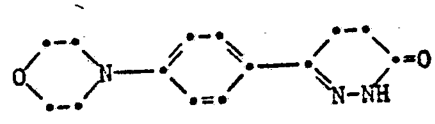

(VII)

18. A process for the preparation of a compound of formula I according to claim 1, wherein R is an amino group, which process comprises

a) solvolysing a compound of formula VIII

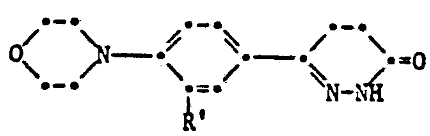

(VIII)

wherein R′ is an amino group protected by a protecting group ; or

b) selectively reducing in a compound of formula IX

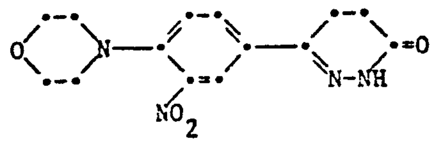

(IX)

the nitro group to the amino group ; and/or, if desired, converting in a resultant compound of formula I, in which R is an amino group, said group into an acetylamino group, and/or, if desired, converting a resultant compound of formula I, in which R is an amino group, into an acid addition salt, and/or, if desired, converting a resultant acid addition salt of a compound of formula I into a compound containing a free amino group.

19. A compound obtainable by a process according to any one of claims 16, 17 and 18.

**Claims** (for the Contracting State AT)

1. A process for the preparation of a compound of the general formula I

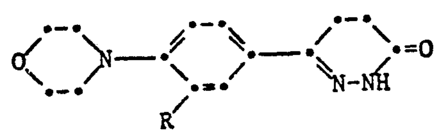

(I)

wherein R is a halogen atom, or the amino, acetylamino, methyl, cyano, methoxy or trifluoromethyl group, which process comprises

a) reacting a ketocarboxylic acid of formula II

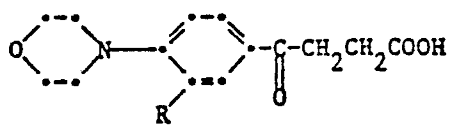

(II)

or a reactive derivative of such a ketocarboxylic acid, wherein R is as defined for formula I, with hydrazine ; or

b) reacting a compound of formula III

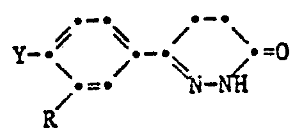

(III)

wherein R is as defined for formula I, and Y is a group which is detachable together with hydrogen, with morpholine ; or

c) reacting a compound of formula IV

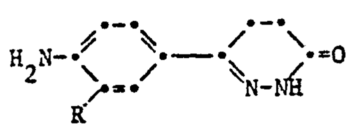

(IV)

with a diethyl ether derivative of formula V

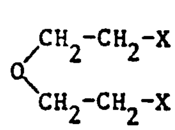

(V)

wherein X is a group which is detachable together with hydrogen ; and/or, if desired, converting in a resultant compound of formula I, in which R is an amino group, said group into an acetylamino group, and/or, if desired, converting a resultant compound of formula I, in which R is an amino group, into an acid addition salt, and/or, if desired, converting a resultant acid addition salt of a compound of formula I into a compound containing a free amino group ; or of a tautomeric form thereof.

2. A process for the preparation of a compound of formula I according to claim 1, wherein R is a halogen atom or a cyano group, which process comprises

a) introducing into a compound of formula VI

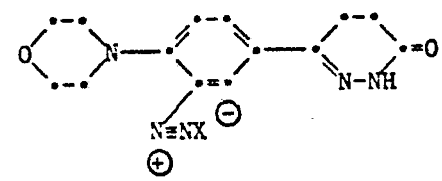

(VI)

wherein $X^{\ominus}$ is an anion of a mineral acid, in the presence of copper or of a copper (I) salt, a halogen atom or a cyano group as R ; or

b) halogenating a compound of formula VII

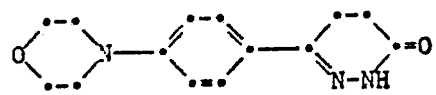

(VII)

3. A process for the preparation of a compound of formula I according to claim 1, wherein R is an amino group, which process comprises

a) solvolysing a compound of formula VIII

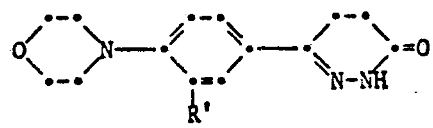

(VIII)

wherein R′ is an amino group protected by a protecting group ; or

b) selectively reducing in a compound of formula IX

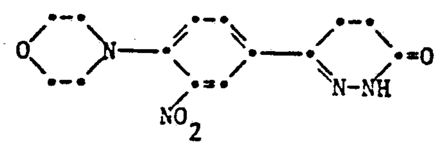

(IX)

the nitro group to the amino group ; and/or, if desired, converting in a resultant compound of formula I, in which R is an amino group, said group into an acetylamino group, and/or, if desired, converting a resultant compound of formula I, in which R is an amino group, into an acid addition salt, and/or, if desired, converting a resultant acid addition salt of a compound of formula I into a compound containing a free amino group.

4. A process for the preparation of a compound of the general formula I

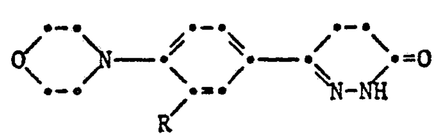

(I)

wherein R is a halogen atom or the trifluoromethyl group, which process comprises

a) reacting a ketocarboxylic acid of formula II

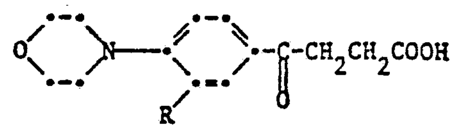

(II)

or a reactive derivative of such a ketocarboxylic acid, wherein R is as defined for formula I, with hydrazine ; or

b) reacting a compound of formula III

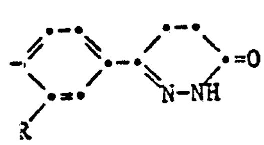

(III)

wherein R is as defined for formula I, and Y is a group which is detachable together with hydrogen, with morpholine, or of a tautomeric form thereof.

5. A process for the preparation of a compound of formula I according to claim 4, which process comprises halogenating a compound of formula VII

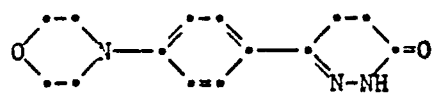

(VII)

6. A process according to claim 5 for the preparation of a compound of formula I, which process comprises effecting the halogenation of a compound of formula VII by heating a compound of formula VI

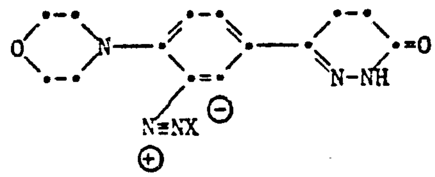

(VI)

wherein $X^{\ominus}$ is an anion of a mineral acid.

7. A process according to either of claims 5 and 6 for the preparation of a compound of formula I, which comprises effecting the halogenation by heating a compound of formula VI, in the presence of copper powder.

8. A process according to claim 1, which comprises preparing a compound of formula I, wherein R is a halogen atom, or the amino, acetylamino, methyl, cyano, methoxy or trifluoromethyl group, or a tautomeric form thereof.

9. A process according to claim 4, which comprises preparing a compound of formula I, wherein R is a halogen atom, or a tautomeric form thereof.

10. A process according to claim 4, which comprises preparing a compound of formula I, wherein R is a chlorine atom, or a tautomeric form thereof.

11. A process according to claim 1, which comprises preparing a compound of formula I, wherein R is an amino group, or a tautomeric form thereof.

12. A process according to claim 1, which comprises preparing a compound of formula I, wherein R is an acetylamino group, or a tautomeric form thereof.

13. A process according to claim 1, which comprises preparing a compound of formula I, wherein R is a cyano group, or a tautomeric form thereof.

14. A pharmaceutical composition containing a compound prepared according to any one of claims 1, 2, 3, 8 and 11 to 13.

15. A pharmaceutical composition containing a compound prepared according to any one of claims 4 to 7, 9 and 12.

**Revendications** (pour les Etats contractants : BE, CH, DE, FR, IT, LI, LU, NL, SE)

1. Pyridazinones de formule générale I

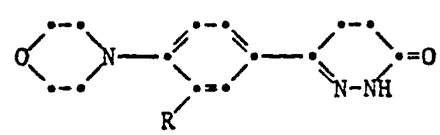

(I)

dans laquelle R représente un atome d'halogène, un groupe amino, acétylamino, méthyle, cyano, méthoxy ou trifluorométhyle, et leurs formes tautomères.

2. Pyridazinones de formule générale I

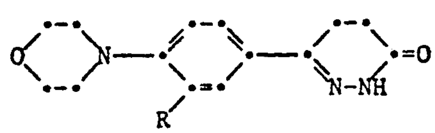

(I)

dans laquelle R représente un atome d'halogène ou le groupe trifluorométhyle, et leurs formes tautomères.

3. Pyridazinones de formule générale I selon la revendication 1, dans laquelle R représente un atome de chlore ou de brome, et leurs formes tautomères.

4. La 6-(3-chloro-4-morpholino-phényl)-4,5-dihydro-3(2H)-pyridazinone et ses formes tautomères.

5. La 6-(3-amino-4-morpholino-phényl)-4,5-dihydro-3(2H)-pyridazinone et ses formes tautomères.

6. La 6-(3-acétylamino-4-morpholino-phényl)-4,5-dihydro-3(2H)-pyridazinone et ses formes tautomères.

7. La 6-(3-cyano-4-morpholino-phényl)-4,5-dihydro-3(2H)-pyridazone et ses formes tautomères.

8. Compositions pharmaceutiques contenant l'un des composés de la revendication 1.

9. Les composés de la revendication 1 pour l'utilisation dans un procédé pour le traitement thérapeutique du corps humain ou animal.

10. Les composés de la revendication 1 pour l'utilisation en tant qu'agents antithrombotiques actifs.

11. Utilisation des composés de la revendication 1 pour la préparation de compositions pharmaceutiques.

12. Compositions pharmaceutiques contenant l'un des composés des revendications 2 à 4.

13. Les composés des revendications 2 à 4 pour l'utilisation dans un procédé pour le traitement thérapeutique du corps humain ou animal.

14. Les composés des revendications 2 à 4 pour l'utilisation en tant qu'agents actifs antithrombotiques.

15. Utilisation des composés des revendications 2 à 4, pour la préparation de compositions pharmaceutiques.

16. Procédé de préparation des composés de formule générale I

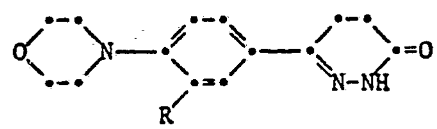

(I)

dans laquelle R représente un atome d'halogène, un groupe amino, acétylamino, méthyle, cyano, méthoxy ou trifluorométhyle, et de leurs formes tautomères, caractérisé en ce que

a) on fait réagir un acide cétocarboxylique de formule II

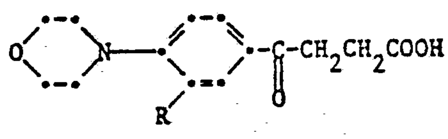

(II)

ou un dérivé réactif d'un tel acide cétocarboxylique, R ayant la signification indiquée en référence à la formule I, avec l'hydrazine, ou bien

b) on fait réagir un composé de formule III

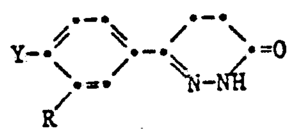

(III)

dans laquelle R a la signification indiquée en référence à la formule I et Y représente, avec l'hydrogène, un groupe éliminable, avec la morpholine, ou bien

c) on fait réagir un composé de formule IV

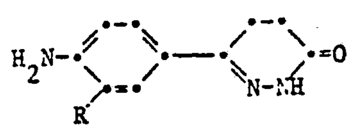

(IV)

avec un dérivé de l'éther diéthylique répondant à la formule V

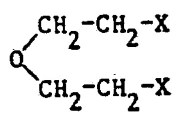

(V)

dans laquelle X représente avec l'hydrogène un groupe éliminable, et/ou, si on le désire, dans un composé obtenu répondant à la formule I dans laquelle R représente un groupe amino, on convertit ce dernier en un groupe acétylamino, et/ou si on le désire, on convertit un composé obtenu répondant à la formule I dans laquelle R représente un groupe amino, en un sel par addition avec un acide, et/ou, si on le désire, on convertit un sel formé par addition avec un acide du composé de formule I, ainsi obtenu, en un composé à groupe amino libre.

17. Procédé de préparation des composés de formule I de la revendication 1, dans laquelle R représente un atome d'halogène ou un groupe cyano, caractérisé en ce que

a) dans un composé de formule VI

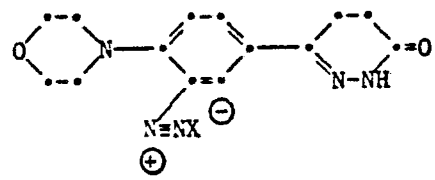

(VI)

dans laquelle $X^{\ominus}$ représente un anion d'un acide minéral, on introduit en tant que substituant R un atome d'halogène ou un groupe cyano en présence de cuivre ou d'un sel cuivreux, ou bien

b) on halogène un composé de formule VII

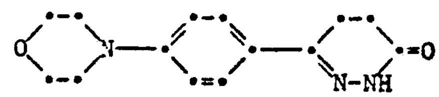

(VII)

18. Procédé de préparation des composés de formule I de la revendication 1 dans laquelle R représente un groupe amino, caractérisé en ce que

a) on soumet un composé de formule VIII

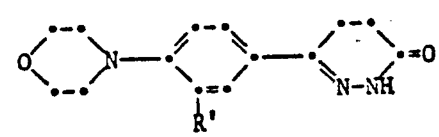

(VIII)

dans laquelle R' représente un groupe amino bloqué par un groupe protecteur, à solvolyse, ou bien

b) dans un composé de formule

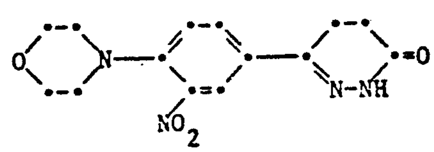

(IX)

on réduit sélectivement le groupe nitro en groupe amino, et/ou, si on le désire, dans un composé obtenu répondant à la formule I dans laquelle R représente un groupe amino, on convertit ce dernier en groupe acétylamino, et/ou, si on le désire, on convertit un composé obtenu répondant à la formule I dans laquelle R représente un groupe amino en un sel par addition avec un acide, et/ou si on le désire, on convertit un composé obtenu consistant en un sel d'un composé de formule I formé par addition avec un acide, en un composé à groupe amino libre.

19. Les composés qu'on peut obtenir par les procédés selon les revendications 16, 17 et 18.

**Revendications** (pour l'Etat contractant AT)

1. Procédé de préparation de composés de formule générale I

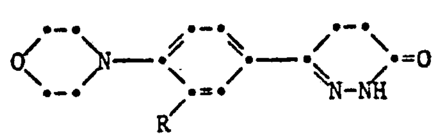

(I)

dans laquelle R représente un atome d'halogène, un groupe amino, acétylamino, méthyle, cyano, méthoxy ou trifluorométhyle, et de leurs formes tautomères, caractérisé en ce que

a) on fait réagir un acide cétocarboxylique de formule II

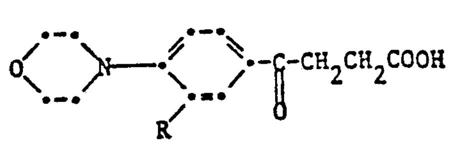

(II)

ou un dérivé réactif d'un tel acide cétocarboxylique, R ayant la signification indiquée en référence à la formule I, avec l'hydrazine, ou bien

b) on fait réagir un composé de formule III

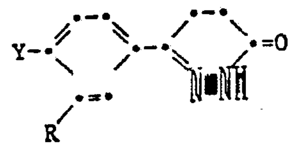

(III)

dans laquelle R a la signification indiquée en référence à la formule I et Y représente avec l'hydrogène un groupe éliminable, avec la morpholine, ou bien

c) on fait réagir un composé de formule IV

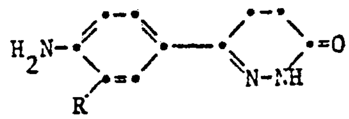

(IV)

avec un dérivé de l'éther diéthylique répondant à la formule V

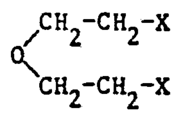

(V)

dans laquelle X représente avec l'hydrogène un groupe éliminable, et/ou, si on le désire, dans un composé obtenu répondant à la formule I dans laquelle R représente un groupe amino, on convertit ce dernier en un groupe acétylamino, et/ou, si on le désire, on convertit un composé obtenu répondant à la formule I dans laquelle R représente un groupe amino, en un sel par addition avec un acide, et/ou, si on le désire, on convertit un composé obtenu, consistant en un sel d'un composé de formule I formé par addition avec un acide, en un composé à groupe amino libre.

2. Procédé de préparation des composés de formule I de la revendication 1, dans laquelle R représente un atome d'halogène ou le groupe cyano, caractérisé en ce que

a) dans un composé de formule VI

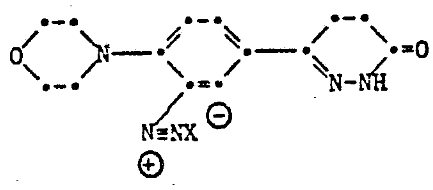

(VI)

dans laquelle $X^-$ représente un anion d'un acide minéral, on introduit en tant que substituant R un atome d'halogène ou un groupe cyano en présence de cuivre ou d'un sel cuivreux, ou bien

b) on halogène un composé de formule VII

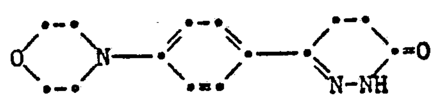

(VII)

3. Procédé de préparation des composés de formule I de la revendication 1, dans laquelle R représente un groupe amino, caractérisé en ce que

a) on soumet un composé de formule VIII

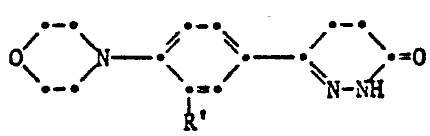

(VIII)

dans laquelle R′ représente un groupe amino bloqué par un groupe protecteur, à solvolyse, ou bien

b) dans un composé de formule

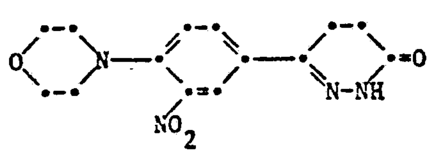

(IX)

on réduit sélectivement le groupe nitro en groupe amino et/ou, si on le désire, dans un composé obtenu répondant à la formule I dans laquelle R représente un groupe amino, on convertit ce dernier en un groupe acétylamino, et/ou si on le désire, on convertit en un sel par addition avec un acide un composé obtenu répondant à la formule I dans laquelle R représente un groupe amino, et/ou, si on le désire, on convertit un composé obtenu, consistant en un sel d'un composé de formule I formé par addition avec un acide, en un composé à groupe amino libre.

4. Procédé de préparation des composés de formule générale I

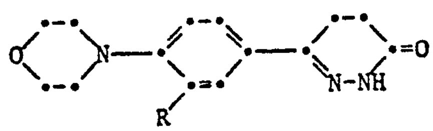

(I)

dans laquelle R représente un atome d'halogène ou le groupe trifluorométhyle, et de leurs formes tautomères, caractérisé en ce que

a) on fait réagir un acide cétocarboxylique de formule II

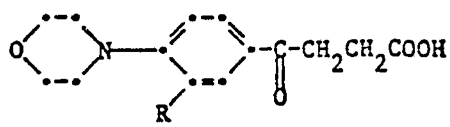

(II)

ou un dérivé réactif d'un tel acide cétocarboxylique, R ayant la signification indiquée en référence à la formule I, avec l'hydrazine,

b) on fait réagir un composé de formule III

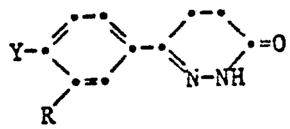

(III)

dans laquelle R a la signification indiquée en référence à la formule I et Y forme avec l'hydrogène un groupe éliminable, avec la morpholine.

5. Procédé de préparation des composés de formule I de la revendication 4, caractérisé en ce que l'on halogène un composé de formule VII

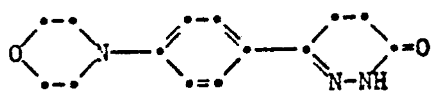

(VII)

6. Procédé selon la revendication 5 pour la préparation des composés de formule I, caractérisé en ce que l'on halogène un composé de formule VII en chauffant un composé de formule VI

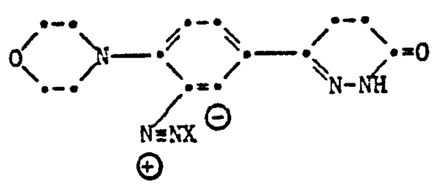

(VI)

dans laquelle $X^{\ominus}$ représente un anion d'un acide minéral.

7. Procédé selon les revendications 5 et 6, pour la préparation des composés de formule I, caractérisé en ce qu'on halogène par chauffage un composé de formule VI en présence de poudre de cuivre.

8. Procédé selon la revendication 1, caractérisé en ce que l'on prépare un composé de formule I et ses formes tautomères, dans lesquels R représente un atome d'halogène, un groupe amino, acétylamino, méthyle, cyano, méthoxy ou trifluorométhyle.

9. Procédé selon la revendication 4, caractérisé en ce que l'on prépare un composé de formule I et ses formes tautomères dans lesquels R représente un atome d'halogène.

10. Procédé selon la revendication 4, caractérisé en ce que l'on prépare un composé de formule I et ses formes tautomères dans lesquels R représente un atome de chlore.

11. Procédé selon la revendication 1, caractérisé en ce que l'on prépare un composé de formule I et ses formes tautomères, dans lesquels R représente un groupe amino.

12. Procédé selon la revendication 1, caractérisé en ce que l'on prépare un composé de formule I et ses formes tautomères, dans lesquels R représente un groupe acétylamino.

13. Procédé selon la revendication 1, caractérisé en ce que l'on prépare un composé de formule I et ses formes tautomères, dans lesquels R représente un groupe cyano.

14. Compositions pharmaceutiques contenant l'un des composés préparés selon les revendications 1, 2, 3, 8 et 11 à 13.

15. Compositions pharmaceutiques contenant l'un des composés préparés selon les revendications 4 à 7, 9 et 12.